# EUROPEAN PATENT APPLICATION

(11) **EP 4 817 663 A1**
(43) Date of publication of application: **07.10.2026**
(21) Application number: 24895818.3
(22) Date of filing: 27.08.2024
(51) Int. Cl.: A61M 16/10

(54) **METHOD AND SYSTEM FOR HUMIDIFYING AND HEATING ADJUSTMENT OF BREATHING MACHINE AND BREATHING MACHINE**

(30) Priority: 27.11.2023 CN 202311591286
(71) Applicant: Heyer Care Co., Ltd., Beijing 100160 (CN)
(72) Inventor: ZENG, Kejun, Beijing 100160 (CN); HU, Ming, Beijing 100160 (CN); HOU, Bingying, Beijing 100160 (CN)
(74) Representative: Bryn Aarflot AS
(86) International application number: PCT/CN2024/114783
(87) International publication number: WO 2025/112717

(57) **Abstract**

A method and system for humidifying and heating adjustment of a breathing machine and a breathing machine. The method comprises: calculating a temperature quantized value of a heating bottom plate of a humidifier according to an environmental temperature, an environmental humidity, and a real-time flow; and using a preset code to determine a humidification level of the humidifier; calculating a heating power quantized value of a heating pipeline according to the real-time flow and the environmental temperature; and using a preset code to determine a heating level of the heating pipeline. According to the method and system for humidifying and heating adjustment and the breathing machine, installation of a sensor at a tail end of the heating pipeline is not required, thereby reducing production costs; the temperature and humidity of the air output by the breathing machine can be automatically adjusted according to different conditions, enabling the breathing machine to better adapt to environmental changes; the breathing machine can also be used when not connected to the heating pipeline, thereby saving costs for a user.

## Description

### CROSS REFERENCE TO RELATED APPLICATION

This application claims priority to Chinese patent application No. 202311591286.0, entitled "Method and System for Humidifying and Heating Adjustment of Breathing Machine and Breathing Machine" filed on November 27, 2023, which is incorporated herein by reference.

### TECHNICAL FIELD

The present application belongs to the field of breathing machines and particularly relates to a method and system for humidifying and heating adjustment of a breathing machine and a breathing machine.

### BACKGROUND

The humidification output of a breathing machine has requirements for temperature and humidity, and a humidification output that does not meet the requirements may cause harm to a user. A humidifier can enable the humidification output to meet the requirements. Currently, most breathing machines adopt a manual manner to set the humidifier level. When therapeutic environmental conditions such as environmental humidity, environmental temperature and real-time flow change, the humidification level needs to be readjusted, which makes the operation cumbersome. Moreover, if the environmental changes are not detected in time, the humidification output may fail to meet the requirements for a long time, thereby causing harm to the user.

In order to solve the above technical problems, existing technical solutions include configuring a heating pipeline for the breathing machine and providing a temperature sensor or a temperature and humidity sensor at a mask end (terminal end) of the heating pipeline. For a heating pipeline provided with a temperature and humidity sensor, fixed target values of temperature and humidity are set. When environmental changes cause a deviation between actual values and the target values, the sensor acquires actual temperature and humidity values in real time, and automatically adjusts a humidification level of a humidifier and a heating level of the heating pipeline according to the deviation, so that the humidification output is stabilized at the target temperature and humidity. For a heating pipeline provided with only a temperature sensor, a correlation among input variables, a terminal temperature of the heating pipeline, and the humidification output is fitted based on actual data. By controlling the terminal temperature of the heating pipeline, indirect control of the humidification output is realized.

The implementation of automatic humidification and heating in the existing technical solutions relies on the sensor disposed at the terminal end of the heating pipeline. In order to avoid generating excessive condensate due to too low environmental temperature, the heating pipeline is often only used when the therapeutic environmental temperature is low. If the heating pipeline is not connected, the existing technology is unable to perform automatic humidification and heating control. In addition, the sensor provided at the terminal end of the heating pipeline increases the cost. For users who do not need to select the heating pipeline, the automatic humidification and heating function cannot be used.

### SUMMARY OF THE INVENTION

The objective of the present application is to overcome the defect that in order to automatically control humidification and heating for an existing breathing machine, a terminal sensor needs to be added, thereby increasing the production cost.

To achieve the above objective, the present application proposes a method for humidifying and heating adjustment of a breathing machine. The method includes:
calculating a temperature quantized value of a heating bottom plate of a humidifier according to an environmental temperature, an environmental humidity, and a real-time flow; and using a preset code to set a humidification level of the humidifier;
calculating a heating power quantized value of a heating pipeline according to the real-time flow and the environmental temperature; and using a preset code to set a heating level of the heating pipeline.

As an improvement of the above method, the method for calculating the temperature quantized value of the heating bottom plate is: ${T}_{hum} = {A}_{1} {Q}_{sensor} + {B}_{1} {T}_{env} + {C}_{1} {Hum}_{env} + {D}_{1} {H}_{t} + {E}_{1}$ where *Tₕᵤₘ* represents a target temperature value of the heating bottom plate of the humidifier; *Qₛₑₙₛₒᵣ* represents the real-time flow; *A*₁ represents a flow coefficient of the humidifier; *Tₑₙᵥ* represents the environmental temperature; *B*₁ represents a temperature coefficient of the humidifier; *Humₑₙᵥ* represents the environmental humidity; *C*₁ represents a humidity coefficient of the humidifier; *Hₜ* represents a humidification capacity; *D*₁ represents a humidification capacity coefficient; and *E*₁ represents a temperature correction coefficient of the humidifier.

As an improvement of the above method, the method for determining the flow coefficient of the humidifier *A*₁, the temperature coefficient of the humidifier *B*₁, the humidity coefficient of the humidifier *C*₁ and the humidification capacity coefficient *D*₁ is:
operating the breathing machine under different environmental temperatures and environmental humidities, adjusting a flow value of the breathing machine and a temperature setting value of the heating bottom plate of the humidifier respectively, measuring the humidification capacity of the breathing machine at each temperature value of the heating bottom plate, and recording the environmental temperature, the environmental humidity, the real-time flow, the temperature of the heating bottom plate, and the humidification capacity;
after multiple testing processes, performing data fitting according to the calculation formula for the temperature quantized value of the heating bottom plate to obtain the values of *A*₁, *B*₁, *C*₁, *D*₁, and *E*₁.

As an improvement of the above method, the method for calculating the heating power quantized value of the heating pipeline is: ${P}_{Tube} = {A}_{2} {Q}_{sensor} + {B}_{2} {T}_{env} + {E}_{2}$ where *P_{Tube}* represents a target power of the heating pipeline; *Qₛₑₙₛₒᵣ* represents the real-time flow; *A*₂ represents a flow coefficient of the heating pipeline; *Tₑₙᵥ* represents the environmental temperature; *B*₂ represents a temperature coefficient of the heating pipeline; and *E*₂ represents a power correction coefficient of the heating pipeline.

As an improvement of the above method, the method for determining the flow coefficient of the heating pipeline *A*₂, the temperature coefficient of the heating pipeline *B*₂, and the power correction coefficient of the heating pipeline *E*₂ is:
under different environmental temperatures and real-time flow conditions, testing the minimum heating pipeline level required to prevent condensation in the pipeline of the breathing machine, and recording the environmental temperature, the real-time flow, and the heating pipeline level during the testing process;
after multiple testing processes, performing data fitting according to the method for calculating the heating power quantized value of the heating pipeline to obtain the values of *A*₂, *B*₂, and *E*₂.

As an improvement of the above method, the method for data fitting is the least squares method, the Lagrange interpolation method, the Newton interpolation method, or a support vector machine-based fitting algorithm.

As an improvement of the above method, the method also includes:
before setting the heating level of the heating pipeline, when the breathing machine is not connected to the heating pipeline, turning off the heating function of the pipeline.

As an improvement of the above method, the method also includes:
when the intelligent humidifying and heating function is not turned on, manually setting the humidification level of the humidifier and the heating level of the heating pipeline.

The present application also provides a system for humidifying and heating adjustment of a breathing machine, which is implemented based on the above method. The system includes:
a humidification level adjustment module for a humidifier, configured to calculate a temperature quantized value of a heating bottom plate of a humidifier according to an environmental temperature, an environmental humidity, and a real-time flow, and use a preset code to determine a humidification level of the humidifier; and
a heating level adjustment module for a heating pipeline, configured to calculate a heating power quantized value of a heating pipeline according to the real-time flow and the environmental temperature, and use a preset code to determine a heating level of the heating pipeline.

The present application also provides a breathing machine, which is implemented based on the above system for humidifying and heating adjustment.

Compared with the prior art, the present application has the following advantages:
1. The intelligent humidifying and heating method provided by the present application does not require the installation of a sensor at a tail end of the heating pipeline, thereby reducing production costs.
2. According to the present application, a correlation between humidification and heating and environmental temperature and environmental humidity is fitted based on historical data, enabling automatic adjustment of the temperature and humidity of the air output by the breathing machine according to different conditions, making the breathing machine better adapt to environmental changes.
3. The intelligent humidifying and heating method provided by the present application can also be used when the heating pipeline is not connected, thereby saving costs for a user.

### BRIEF DESCRIPTION OF THE DRAWINGS

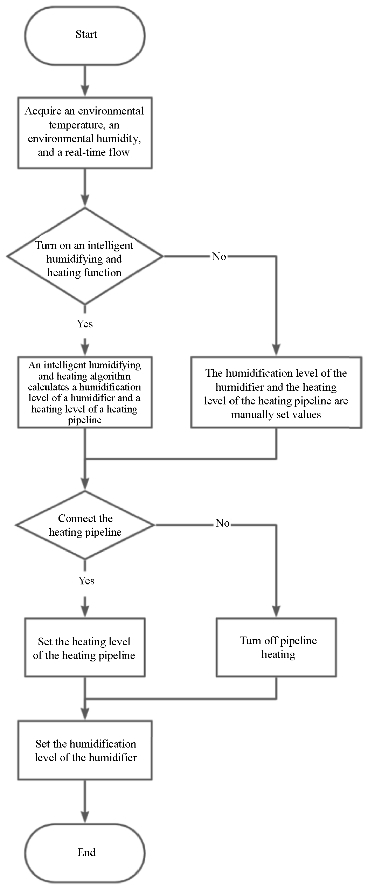

Fig. 1 shows a flow diagram of a method for humidifying and heating adjustment of a breathing machine.

### DETAILED DESCRIPTION

The technical solutions of the present application will be described in detail below in conjunction with the accompanying drawings.

The present application provides a method and system for humidifying and heating adjustment of a breathing machine and a breathing machine. The humidifying and heating algorithm provided by this method can automatically adjust the humidification level of the humidifier and the heating level of the heating pipeline according to the environmental temperature, the environmental humidity and the real-time flow. The breathing machine provided by the present application does not require a sensor at the tail end of the heating pipeline, thereby reducing the cost. Moreover, the intelligent humidifying and heating function can also be realized without connecting the heating pipeline, and users do not have to select a heating pipeline solely for the intelligent humidifying and heating function.

During operation of the breathing machine, the environmental temperature, the environmental humidity, and the real-time flow are acquired in real time (the real-time flow refers to the flow value measured by the flow sensor of the breathing machine in real time, which measures the air flow inhaled at the air inlet of the breathing machine, not just the oxygen supply flow). During therapy with the breathing machine, it is determined whether the intelligent humidifying and heating function is turned on. If not turned on, the breathing machine adjusts the humidifier and the heating pipeline according to manually set values. If turned on, the temperature quantized value of the heating bottom plate of the humidifier is calculated based on the intelligent humidifying and heating algorithm, and the humidification level of the humidifier is determined by means of the preset code. The specific algorithm is: ${T}_{hum} = {A}_{1} {Q}_{sensor} + {B}_{1} {T}_{env} + {C}_{1} {Hum}_{env} + {D}_{1} {H}_{t} + {E}_{1}$ (1) where *Tₕᵤₘ* is a target temperature value of the heating bottom plate of the humidifier; *Qₛₑₙₛₒᵣ* is the real-time flow; *A*₁ is a flow coefficient of the humidifier; *Tₑₙᵥ* is the environmental temperature; *B*₁ is a temperature coefficient of the humidifier; *Humₑₙᵥ* is the environmental humidity; *C*₁ is a humidity coefficient of the humidifier; *Hₜ* is the humidification capacity; *D*₁ is a humidification capacity coefficient; and *E*₁ is a temperature correction coefficient of the humidifier.

During therapy with the breathing machine, it is determined whether a heating pipeline is connected. If the heating pipeline is not connected, the pipeline heating is turned off. If the heating pipeline is connected, the heating power quantized value of the heating pipeline is calculated according to the intelligent humidifying and heating algorithm, and the heating level of the heating pipeline is determined according to the preset code. The specific algorithm is: ${P}_{Tube} = {A}_{2} {Q}_{sensor} + {B}_{2} {T}_{env} + {E}_{2}$ (2) where *P_{Tube}* is a target power of the heating pipeline; *Qₛₑₙₛₒᵣ* is the real-time flow; *A*₂ is a flow coefficient of the heating pipeline; *Tₑₙᵥ* is the environmental temperature; *B*₂ is a temperature coefficient of the heating pipeline; and *E*₂ is a power correction coefficient of the heating pipeline.

The coefficients of the above intelligent humidifying and heating algorithm are determined or modified based on the conditions of the system. Here, a method for determining the algorithm coefficients is given, and the determination of the algorithm coefficients is not limited to this method. The breathing machine operates under different environmental temperatures and environmental humidities. The flow of the breathing machine is set to a fixed value, and the temperature setting value of the heating bottom plate of the humidifier is adjusted respectively, and the humidification capacity of the breathing machine is measured at each temperature value of the heating bottom plate. Then the flow value is changed, and the temperature value of the heating bottom plate of the humidifier is adjusted respectively again to measure the humidification capacity. The above tests are repeated a certain number of times. During the testing process, parameters such as the environmental temperature, the environmental humidity, the real-time flow, the temperature of the heating bottom plate, and the humidification capacity are recorded. Data fitting is performed according to the intelligent humidification algorithm, thereby obtaining the specific values of *A*₁ , *B*₁ , *C*₁ , *D*₁ , and *E*₁ . Under different environmental temperatures and real-time flows, the minimum heating pipeline level required to prevent condensation in the pipeline of the breathing machine is tested, and the environmental temperature, the real-time flow, and the heating pipeline level are recorded during the testing process. Data fitting is performed according to the intelligent humidification algorithm to obtain the specific values of *A*₂, *B*₂, and *E*₂. The data fitting method may adopt the least squares method, the Lagrange interpolation method, the Newton interpolation method, or a support vector machine-based fitting algorithm.

As shown in Fig. 1, the process of the intelligent temperature and humidity adjustment in the breathing machine includes: the breathing machine acquires the environmental temperature, the environmental humidity, and the real-time flow in real time. If the intelligent humidifying and heating function is enabled, the target humidification capacity, the environmental temperature, the environmental humidity, and the real-time flow of the breathing machine are substituted into formula (1) to calculate the temperature of the heating bottom plate (the humidification level), with the breathing machine controlling the heating bottom plate to reach the temperature value solved by the algorithm formula. The environmental temperature and the real-time flow are substituted into formula (2) to calculate the heating level of the heating pipeline, with the breathing machine controlling the heating pipeline to reach the heating level calculated by the algorithm formula. When the intelligent humidifying and heating algorithm in not used, the humidification level and the heating pipeline level of the breathing machine need to be manually set by the user. When the level is set improperly, the humidification capacity output of the breathing machine may fail to reach the set value, and condensate may even appear in the pipeline. When the intelligent humidifying and heating algorithm is used, the breathing machine automatically adjusts the humidifier level and the heating pipeline level to achieve the set humidification capacity output and prevent condensation in the pipeline.

The present application also provides a system for humidifying and heating adjustment of a breathing machine, which is implemented based on the above method. The system includes:
a humidification level adjustment module for a humidifier, configured to calculate a temperature quantized value of a heating bottom plate of a humidifier according to an environmental temperature, an environmental humidity, and a real-time flow, and use a preset code to determine a humidification level of the humidifier; and
a heating level adjustment module for a heating pipeline, configured to calculate a heating power quantized value of a heating pipeline according to the real-time flow and the environmental temperature, and use a preset code to determine a heating level of the heating pipeline.

The present application also provides a breathing machine, which is implemented based on the above system for humidifying and heating adjustment.

Finally, it should be noted that the above embodiments are only used for describing instead of limiting the technical solutions of the present application. Although the present application has been described in detail with reference to the embodiments, persons of ordinary skill in the art should understand that modifications or equivalent substitutions of the technical solutions of the present application should be encompassed within the scope of the claims of the present application so long as they do not depart from the spirit and scope of the technical solutions of the present application.

## Claims

1. A method for humidifying and heating adjustment of a breathing machine, comprising:
calculating a temperature quantized value of a heating bottom plate of a humidifier according to an environmental temperature, an environmental humidity, and a real-time flow; and using a preset code to set a humidification level of the humidifier;
calculating a heating power quantized value of a heating pipeline according to the real-time flow and the environmental temperature; and using a preset code to set a heating level of the heating pipeline.

2. The method for humidifying and heating adjustment of a breathing machine according to claim 1, wherein the method for calculating the temperature quantized value of the heating bottom plate is: ${T}_{hum} = {A}_{1} {Q}_{sensor} + {B}_{1} {T}_{env} + {C}_{1} {Hum}_{env} + {D}_{1} {H}_{t} + {E}_{1}$ where *Tₕᵤₘ* represents a target temperature value of the heating bottom plate of the humidifier; *Qₛₑₙₛₒᵣ* represents the real-time flow; *A*₁ represents a flow coefficient of the humidifier; *Tₑₙᵥ* represents the environmental temperature; *B*₁ represents a temperature coefficient of the humidifier; *Humₑₙᵥ* represents the environmental humidity; *C*₁ represents a humidity coefficient of the humidifier; *Hₜ* represents a humidification capacity; *D*₁ represents a humidification capacity coefficient; and *E*₁ represents a temperature correction coefficient of the humidifier.

3. The method for humidifying and heating adjustment of a breathing machine according to claim 2, wherein the method for determining the flow coefficient of the humidifier *A*₁, the temperature coefficient of the humidifier *B*₁, the humidity coefficient of the humidifier *C*₁, and the humidification capacity coefficient *D*₁ is:
operating the breathing machine under different environmental temperatures and environmental humidities, adjusting a flow value of the breathing machine and a temperature setting value of the heating bottom plate of the humidifier respectively, measuring the humidification capacity of the breathing machine at each temperature value of the heating bottom plate, and recording the environmental temperature, the environmental humidity, the real-time flow, the temperature of the heating bottom plate, and the humidification capacity;
after multiple testing processes, performing data fitting according to the calculation formula for the temperature quantized value of the heating bottom plate to obtain the values of *A*₁, *B*₁, *C*₁, *D*₁, and *E*₁.

4. The method for humidifying and heating adjustment of a breathing machine according to claim 1, wherein the method for calculating the heating power quantized value of the heating pipeline is: ${P}_{Tube} = {A}_{2} {Q}_{sensor} + {B}_{2} {T}_{env} + {E}_{2}$ where *P_{Tube}* represents a target power of the heating pipeline; *Qₛₑₙₛₒᵣ* represents the real-time flow; *A*₂ represents a flow coefficient of the heating pipeline; *Tₑₙᵥ* represents the environmental temperature; *B*₂ represents a temperature coefficient of the heating pipeline; and *E*₂ represents a power correction coefficient of the heating pipeline.

5. The method for humidifying and heating adjustment of a breathing machine according to claim 4, wherein the method for determining the flow coefficient of the heating pipeline *A*₂, the temperature coefficient of the heating pipeline *B*₂ and the power correction coefficient of the heating pipeline *E*₂ is:
under different environmental temperatures and real-time flow conditions, testing the minimum heating pipeline level required to prevent condensation in the pipeline of the breathing machine, and recording the environmental temperature, the real-time flow, and the heating pipeline level during the testing process;
after multiple testing processes, performing data fitting according to the method for calculating the heating power quantized value of the heating pipeline to obtain the values of *A*₂, *B*₂, and *E*₂.

6. The method for humidifying and heating adjustment of a breathing machine according to claim 3 or 5, wherein the method for data fitting is the least squares method, the Lagrange interpolation method, the Newton interpolation method, or a support vector machine-based fitting algorithm.

7. The method for humidifying and heating adjustment of a breathing machine according to claim 1, wherein the method also comprises:
before setting the heating level of the heating pipeline, when the breathing machine is not connected to the heating pipeline, turning off the heating function of the pipeline.

8. The method for humidifying and heating adjustment of a breathing machine according to claim 1, wherein the method also comprises:
when the intelligent humidifying and heating function is not turned on, manually setting the humidification level of the humidifier and the heating level of the heating pipeline.

9. A system for humidifying and heating adjustment of a breathing machine, implemented based on any of the methods of claims 1-8, wherein the system comprises:
a humidification level adjustment module for a humidifier, configured to calculate a temperature quantized value of a heating bottom plate of the humidifier according to an environmental temperature, an environmental humidity, and a real-time flow, and use a preset code to determine a humidification level of the humidifier; and
a heating level adjustment module for a heating pipeline, configured to calculate a heating power quantized value of the heating pipeline according to the real-time flow and the environmental temperature, and use a preset code to determine a heating level of the heating pipeline.

10. A breathing machine implemented based on the humidifying and heating adjustment system of claim 9.
